# EUROPEAN PATENT SPECIFICATION

(11) **EP 0 469 129 B1**
(45) Date of publication and mention of the grant of the patent: **04.11.1998**
(21) Application number: 91904929.6
(22) Date of filing: 18.02.1991
(51) Int. Cl.: A61K 31/045, A61K 31/075, B29C 45/00, B29C 47/00

(54) **An infection resistant article and method of making compounds for their manufacture**
Infektionsresistente Gegenstände und Methode zur Herstellung von Zusammensetzungen zu deren Anfertigung
Articles résistants aux infections et méthode pour la préparation de composés pour la fabrication desdits articles.

(30) Priority: 22.02.1990 US 484137
(43) Date of publication of application: 05.02.1992
(73) Proprietor: Allegiance Corporation, McGaw Park, Illinois 60085-6787 (US)
(72) Inventor: KERSTEIN, Jean, B-7931 Villers-S.-Amand (BE); DELMOTTE, Yves, A., B-7333 Tertre (BE)
(74) Representative: MacGregor, Gordon
(86) International application number: US9101045
(87) International publication number: WO9112796

(56) References cited:
- EP-A- 0 305 551
- WO-A-90/14048
- US-A- 4 040 997
- US-A- 4 544 694
- US-A- 4 581 028
- DATABASE WPI, Week 8220, Derwent Publications Ltd., London, GB; AN 40680E & JP-A-57 061 036 13 April 1982

## Description

### BACKGROUND OF THE INVENTION

### Field of the Invention

The invention relates to polymeric compound to articles made from, or coated with an infection resistant compound.

### The Prior Art

It is known that, for manufacturing articles from a molten blend of a polymer, it is suitable to add some additives such as plasticizer(s) and antioxidant(s).

It is also known to cover articles with a layer containing an antibactetial agent. For covering such articles, a composition containing a polymer, a solvent of said polymer and an antibacterial agent is prepared, the composition is then applied on the surface of the device so that, after drying, the article is provided with an antibacterial polymeric layer. Such articles are expensive and have antibacterial properties only on one surface thereof.

If is known to add to molten polyvinyl chloride benzoate of sodium or mercury salts for avoiding a microbial growth on said polymer. However, such additives are toxic so that the use thereof has to be proscribed.

Nonoxynol-9, a non-ionic surfactant of the general formula 1, has been described as an inhibitor of the growth of several microbes such as herpes simplex virus, HTLV-III. Nonoxynol-9 has already been used in spermacides.

Applicants have found that compounds of the general formula 1: 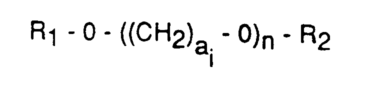 are antiviral agents, plasticizers and antioxidants, which are stable at temperatures higher than 100°C, so that they may be used in usual injection, extrusion, or other transformation processes.

### SUMMARY OF THE INVENTION

The invention relates to an article made from or coated with a layer of a plasticized polymeric compound, the plasticizer including, in an amount of at least 1 % of the compound, an agent of the general formula:

R₁ - O - ((CH₂)aᵢ - O)n - R₂

where:
R₁ is a substituted cycloalkyl or substituted aromatic radical;
aᵢ is, for i = 1 to n, an integer greater or equal to 2;
R₂ is hydrogen or a hydrocarbon radical; and
n is an integer selected so that the HLB of said compound is comprised between 12 and 20
to provide antioxidant and antiviral properties and reduce gas permeability of the article, the article not being a vaginal device.

The compound may be: extruded, injected or dip moulded so as to manufacture infection-resistant articles; or sprayed on articles so as to provide said articles with an infection-resistant layer.

The articles may be, for example, surgical gloves, surgical clothes, surgical operative fields, finger stalls, aprons, bibs and caps.

The invention also relates to such articles made from or coated with a layer of said compound.

### BRIEF DESCRIPTION OF THE DRAWINGS

Figures 1 to 8 are chromatograms at 225, 254 and 271 nm of agents suitable for the invention;
Figure 9 is a UV spectrum of an agent suitable for the invention;
Figures 10 to 12 show the stability of polyvinylchloride at 175°C; and
Figures 13 and 14 show permeabilities of polyvinylchloride.

### DETAILED DESCRIPTION OF THE PREFERRED EMBODIMENT

Compounds of general formula 1: 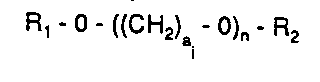 are known as being non-ionic surfactants.

Said compound may be characterized by a hydrophile-lipophile balance (HLB) as taught by Griffin, W. C., ,**J. Soc. Cosmet. Chem.1,** (1949) 311-326.

Such compounds are, for example, alkylphenoxypoly (ethyleneoxy) ethanol and more specifically nonylphenoxypoly (ethyleneoxy) ethanol (Antarox®, Antarox®CO-630, Nonoxynol -9).

Methods for the manufacture of such compounds are given for example, in U.S. Patent No. 1,970,578 and U.S. Patent No. 2,774,709.

It has been found that said compounds when treated at temperatures higher than 100°C have always virucide action for example against Hepatitis A, Hepatitis B and AIDS (HTL VIII). The compounds were even stable at temperature of about 200°C.

In a method of producing an article according to the invention, such a compound is mixed with a plasticizer before being added to a molten polymer. When manufacturing for example disposable gloves, the compound will be distributed between the surface and the polymer matrix. Therefore, the infection-resistant articles provide an antiviral contact protection and a protection mechanism in case of pinholes or microcracks.

Since the compounds do not affect the polymer network properties, such as tensile strength, elasticity modules, etc. up to 10% or even more of said compounds may be added.

Moreover, since said compounds have a similar plasticising effect as known plasticizers such as di iso nonyl phthalate and (DINP), dialkylhexylphthalate, the compounds may replace the plasticizers.

The compound has preferably the following characteristics:
- R₁: is a substituted cycloalkyl or aromatic radical advantageously a phenyl radical substituted by at least one alkyl group and preferably a phenyl radical substituted in para position with a C₆₋₁₂ alkyl group;
- aᵢ: is 2 or 3;
- n: is an integer comprised between 6 and 20, preferably between 8 and 15; and
- R₂: is a radical selected from hydrogen and alcohol.

Polymers which may be used are polyethylene (low density, high density), polypropylene, polyvinylchloride, polystyrene, polyamide, polycarbonate and rubber.

The C₆₋₁₂ alkyl group which is used as substituent in para position of the phenyl radical may be selected from linear alkyl group such as n-hexyl, n-heptyl, n-octyl, n-nonyl, n-decyl, n-undecyl and n-dodecyl and branched alkyl group such as methyl pentyl, methyl heptyl, methyl octyl, methyl nonyl, methyl decyl, methyl undecyl, di-methyl hexy], 4-(tert-octyl), ethyl propyl.

Preferred alkyl groups are n-octyl, n-nonyl, 4-(tert octyl), 7-methylheptyl, 6-dimethylhexyl, 8-methyloctyl, 7-dimethylheptyl.

### EXAMPLES AND TESTS

### Example of Compounds

Compounds which are suitable for the method according to the invention are compounds of formula

R₁ - 0 - ((CH₂)₂ - O)ₙ - H

in which
R₁ 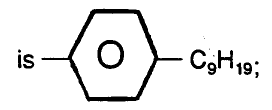 and
n is an integer comprised between 7-8 and 15.

Such compounds are given hereafter:

| **TRADEMARK** | **n** | **HLB** | **Figures** |
|---|---|---|---|
| ANTAROX®CO-610 (sold by GAF CHEMICAL CORP.,U.S.A.) | 7-8 | 12.2 | 1 |
| ANTAROX® CO-620 | 8-9 | 12.6 | 2 |
| ANTAROX® CO-630 | 9 | 13.0 | 3 and 4 |
| ANTAROX® CO-660 | 10 | 13.2 | 5 |
| ANTAROX® CO-710 | 10-11 | 13.6 | 6 |
| ANTAROX® CO-720 | 12 | 14-2 | 7 |
| ANTAROX® CO-730 | 15 | 15 | 8 |

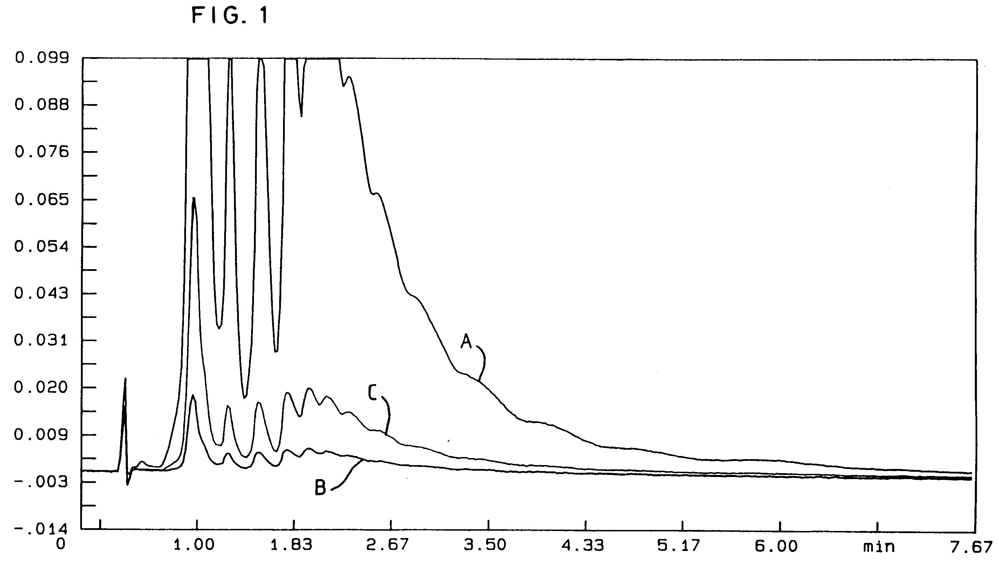

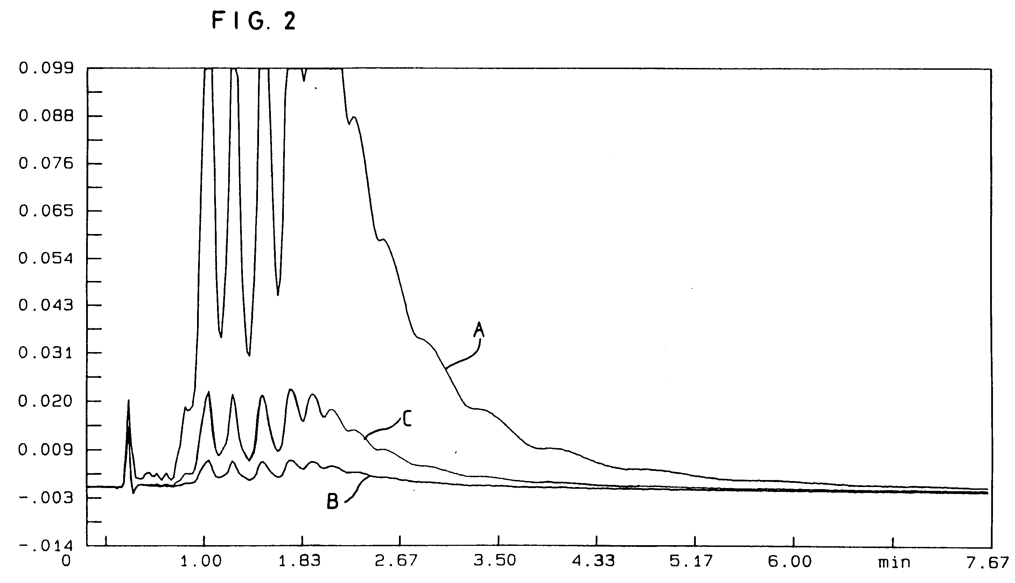

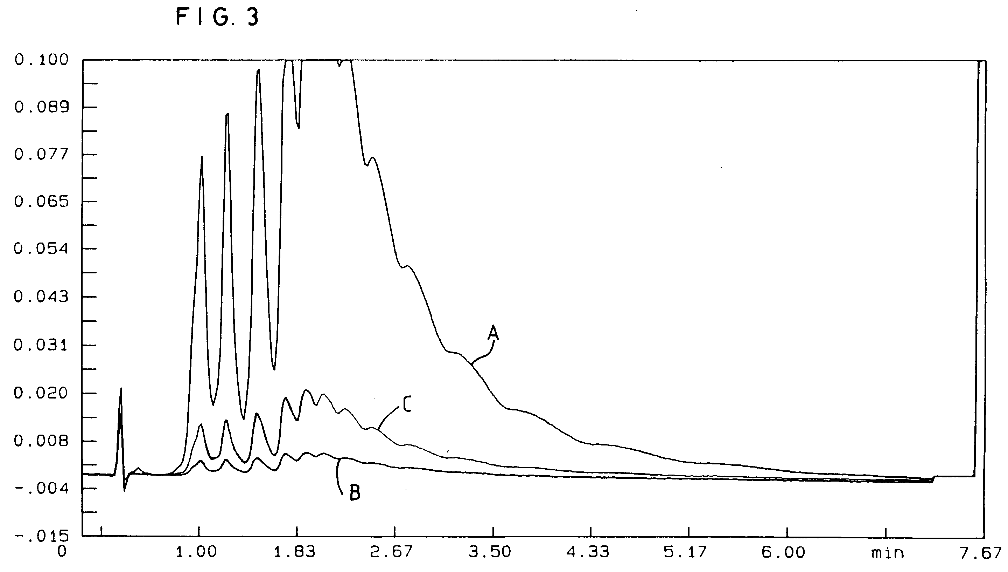

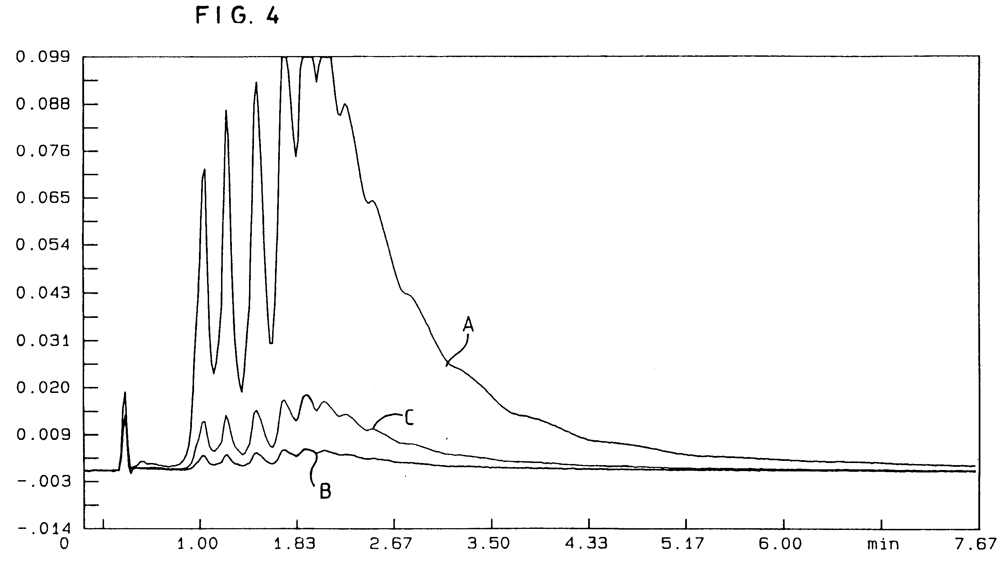

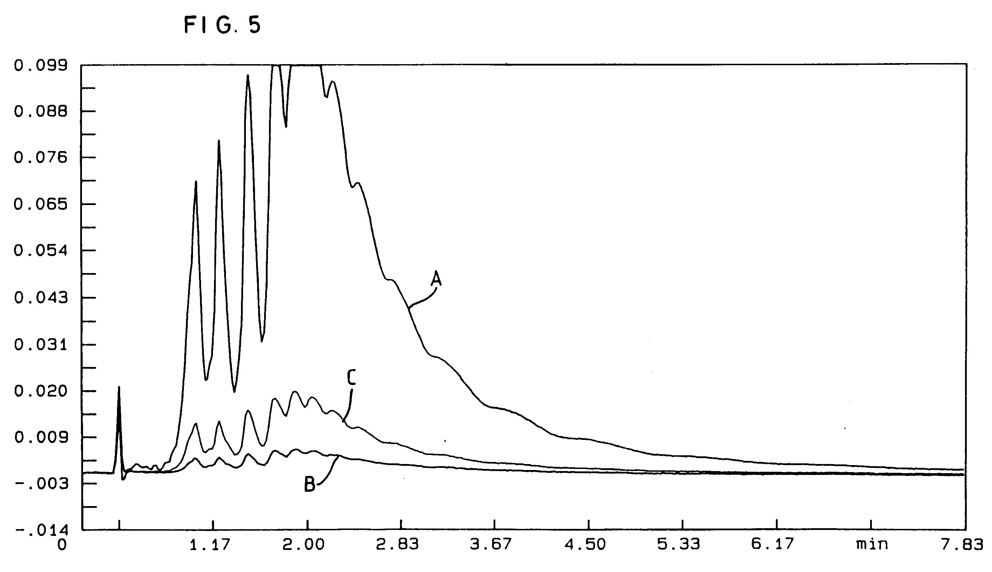

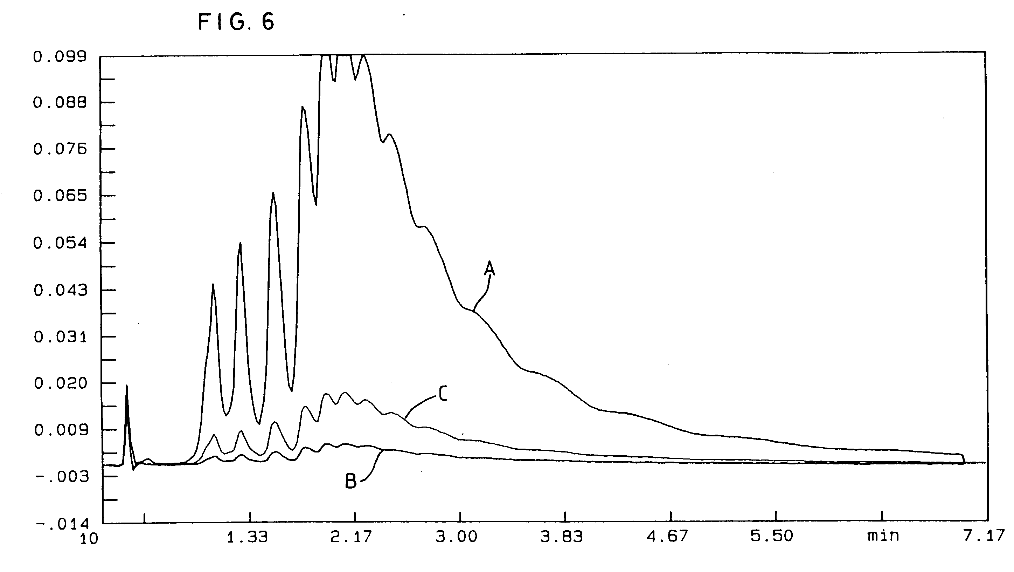

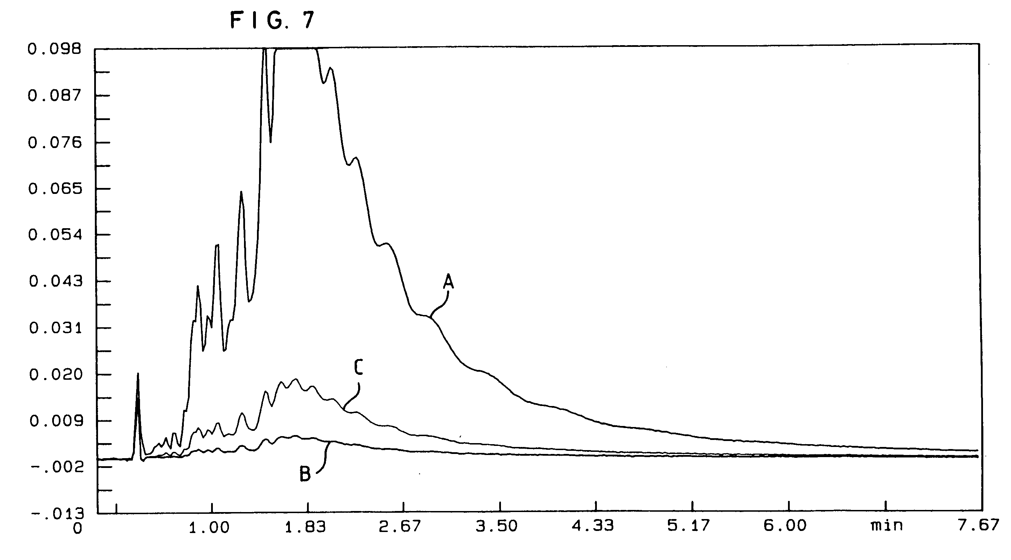

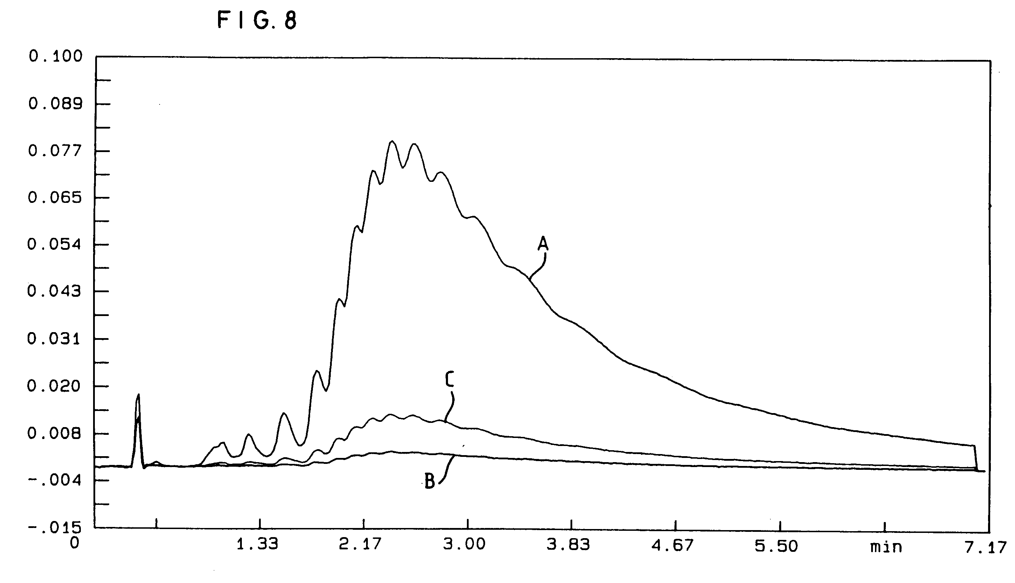

The chromatograms of said compounds are given in figures 1 to 8. In said figures, curve A is the chromatogram at 225 nm, curve B is the chromatogram at 254 nm and curve C is the chromatogram at 271 nm.

It appears from these figures that peaks appear for each compound after about 1.3, 1.6, 1.7, 1.9 and 2.1 minutes.

From this chromatograms, it is clear that the products of the ANTAROX family may be considered as a mixture of various compounds, n being only a mean value.

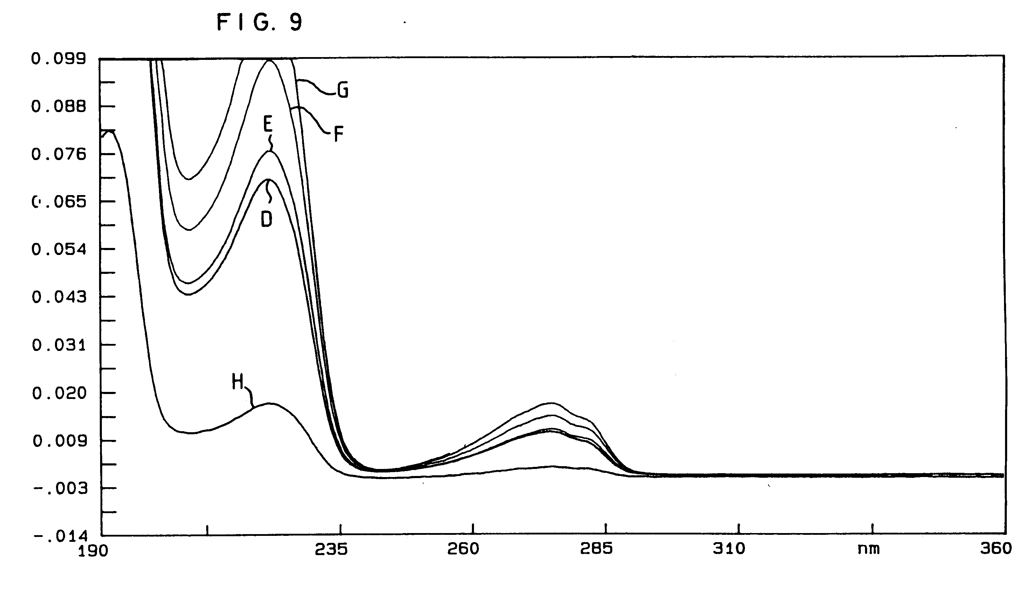

ANTAROX® CO-630 (normal grade) has been analyzed via a UV spectrum (see figure 9). In said figure 9, curves D, E, F, G and H represent the UV spectrum of the fraction of the compound ANTAROX appearing on the chromatogram at respectively 1.04, 1.26, 1.52, 1.78 and 3.78 minutes.

It appears from this UV analysis that ANTAROX CO-630 contains various compounds having the following structure 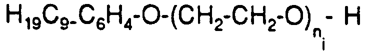 and that the n value given to ANTAROX CO-630 is a mean value of the various nᵢ.

Another example of non ionic surfactant of the formula 1 which is suitable for the invention is: 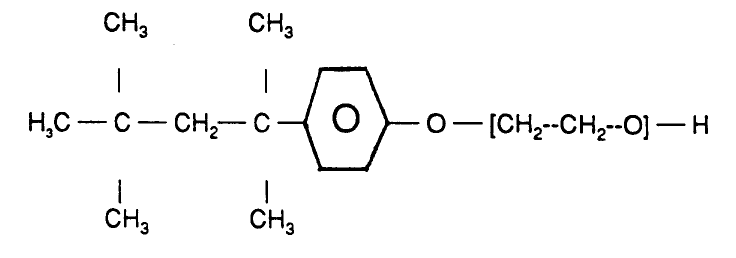 n being equal to 9-10.

Said compound is sold by BOEHRINGER MANNHEIM LABORATORIES under the name TRITON® X - 100.

It is clear that mixture of compounds of general formula 1 may be used as antivirus agent, plasticizer and antioxidant.

### Thermal Stability Tests

The stability of blends containing compounds ANTAROX® CO-630 (normal grade) has been shown by means of the following test.

A sample of polyvinyl chloride has been heated on the following manner:

| Heating time | Temperature and conditions |
|---|---|
| 0 to 3 minutes | the sample is heated at 55°C under nitrogen |
| from 3 minutes to 6 minutes | the temperature of the sample under nitrogen is increased at a rate of 40°C per minutes |
| from 6 minutes to 46 minutes (end of the test) | the temperature of the sample is maintained at 175°C and a flow of oxygen is applied on the sample, the thermal response of which is studied by means of a Perkin Elmer Thermal Analysis System |

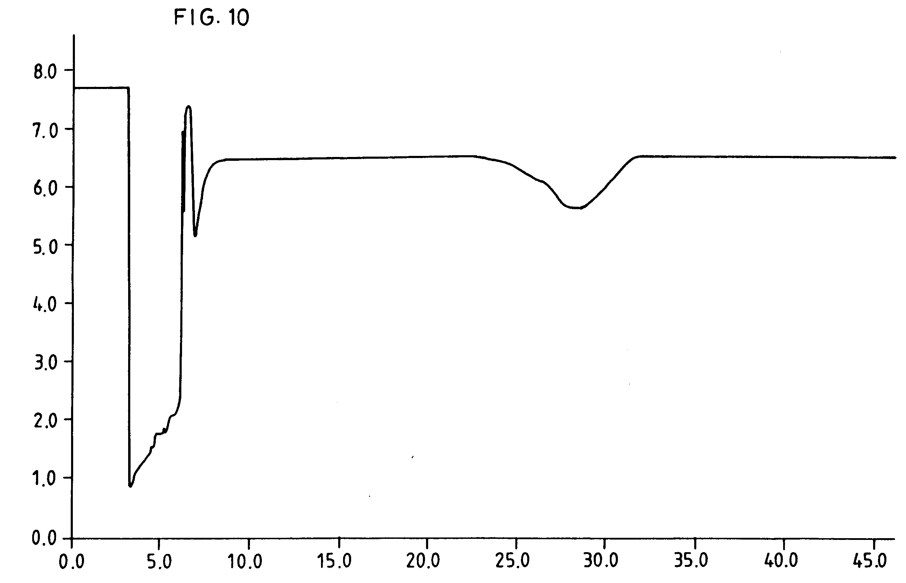

Figures 10 to 12 show the thermal response of PVC sample, i.e., the heat flow (mW) required for heating the sample (P) or for the melting thereof (Q) or for maintaining a temperature of 175°C (R) after a time of X minutes.

If a variation of heat flow occurs while maintaining a temperature of 175°C, it means that the sample is oxidized or degradated.

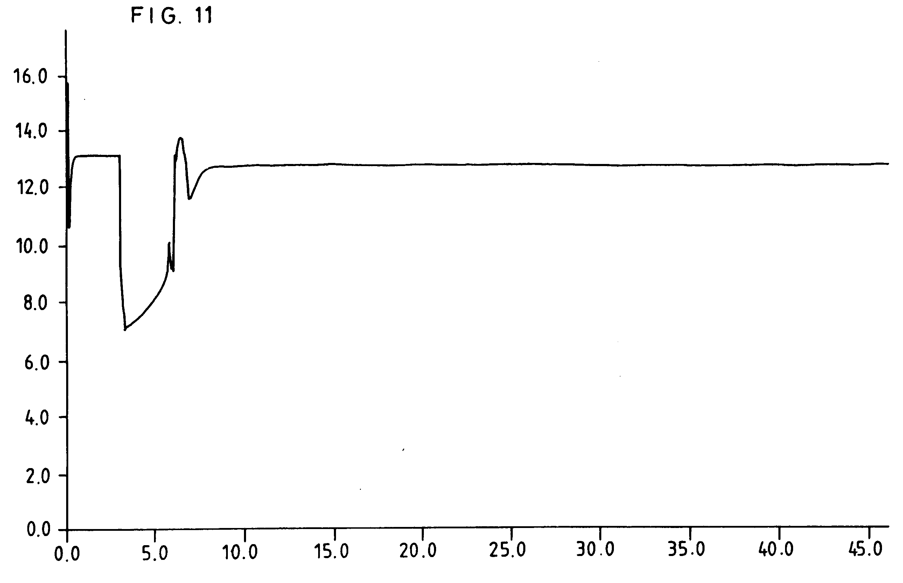

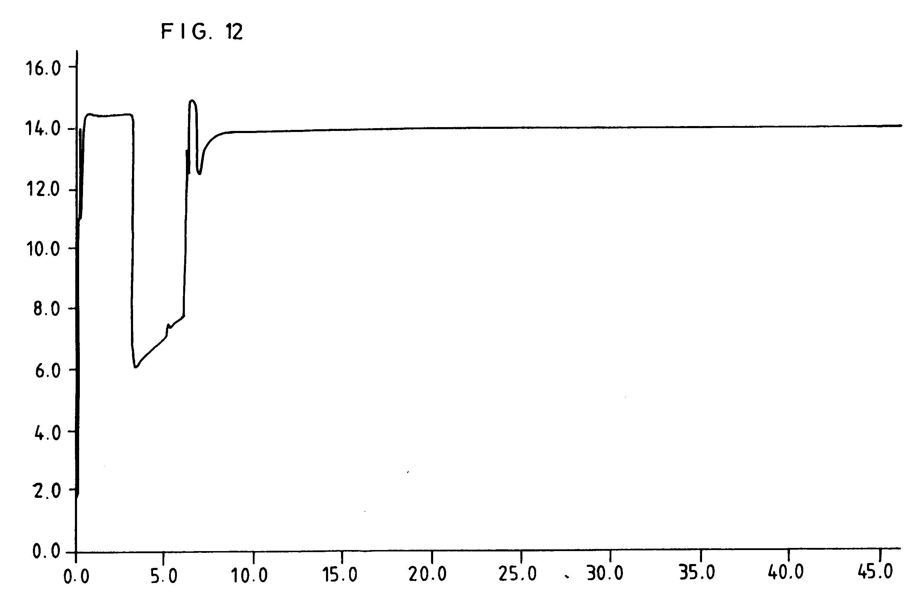

Figure 10 shows the termal response of a PVC sample, with the standard plasticizer (DINP) while Figure 11 and Figure 12 show the influence of replacing the standard plasticizer with an equivalent amount of ANTAROX® CO-630, respectively for 5 and 10% of the formulation concentration.

It appears from these figures that the addition of ANTAROX® CO-630 increases the stability of the PVC sample. It means also that ANTAROX® CO-630 is stable at temperature of 175°C so that said compound may be used in usual techniques such as polymer injection or extrusion without any degradation.

### Examples of Manufacture

Ninety-five kg of liquid plastisol (a blend containing 45% of polyvinyl chloride, the rest essentially DINP) is mixed with 5 kg of ANTAROX® CO-630. Said composition has been cast so as to produce film samples for mechanical tests, after gelification at 175°C.

During the mixing of ANTAROX® CO-630, with the plastisol, no phase separation was observed. The tensile strength of samples was estimated by tests performed with a rate of 200 mm/min. A surface migration of ANTAROX® as observed for the films, the thickness of which was approximative 130 microns.

In a same manner, films samples were manufactured from a composition containing 90 kg of plastisol and 10 kg of ANTAROX® CO-630. A surface migration was also observed for the gloves, the thickness of which was about 130 microns.

The mechanical properties of the casted film samples are summarized in the following table. For comparison purpose said table contains also mechanical properties of samples made only from plastisol.

**TABLE**

| | plastisol | plastisol + 5% ANTAROX | plastisol + 10% ANTAROX |
|---|---|---|---|
| Tensile Strength N/mm² | 8.1 | 6.94 | 5.9 |
| Elongation % | 217 | 241 | 243 |

When using gloves containing ANTAROX® CO-630, no allergic reaction of the users was observed.

### Further Advantage

The gas permeability of polyvinylchloride with or without Antarox CO-630 has also been studied. For this study, samples are set in a measuring chamber comprising two parts, the lower of which being the circulation and sampling system while the upper part is the gassing chamber. The working gas was constituted of respectively one-third of carbon dioxide (CO₂), one-third of oxygen (O₂) and one-third of nitrogen (N₂), said gas flowing in the upper part. The gas passes through the PVC film and is periodically swept by helium gas (vector gas) to the detector where it is quantified.

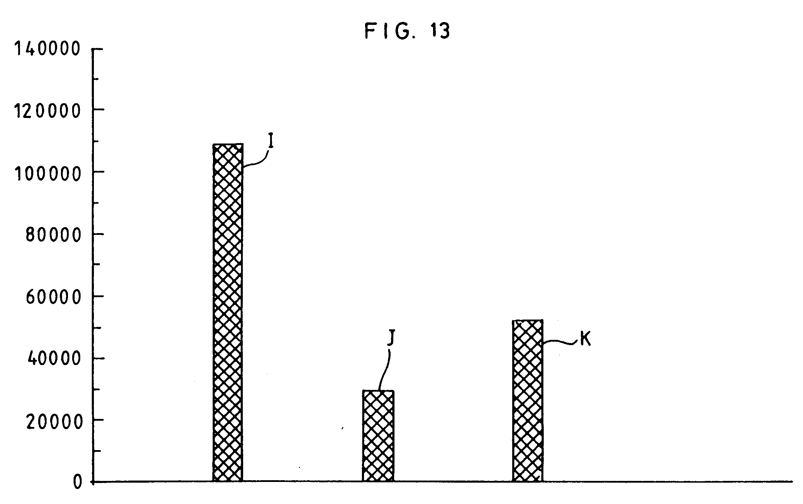

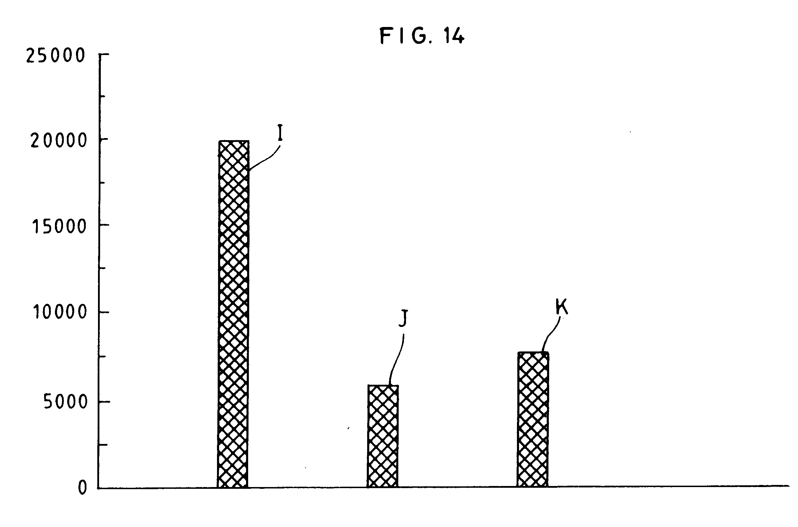

The permeabilities of the films without ANTAROX (film 1, plastisol), of the film containing 5% Antarox CO-630 (film J) and of the film containing 10% Antarox CO-630 (film K) are given in figure 13 for CO₂ and in figure 14 for O₂, said permeabilities being expressed in cm³/day m² atm.

Articles according to the invention are thus effective as a gas barrier.

The replacement of known plasticizer such as DINP by Antarox CO-630 allow to reduce the gas permeability. Therefore, it seems that articles according to the invention have pinholes, the dimensions of which or the number of which are lower than articles which do not contain a compound of formula 1 in the matrix of the polymer. In other words, articles according to the invention have a low porosity.

The gas barrier property of articles according to the invention are improved when the composition contains at least 1% of compound(s) of formula 1, preferably between 2 and 10% and more preferably about 5%.

The viscosity of plastisol is about 800 to 1300 cps at temperature comprised between 30 and 35°C while the viscosity of plastisol containing 10% Antarox® CO-630 is about 920 cps at 32°C. Therefore, the use of compounds of formula 1, in particular of Antarox® CO-630 instead of known plasticizers such as DINP, does not require modification of the manufacture process.

## Claims

1. An article made from or coated with a layer of a plasticized polymeric compound, the plasticizer including, in an amount of at least 1% of the compound, an agent of the general formula:
R₁ - O - ((CH₂))aᵢ - O)n - R₂
where:
R₁ is a substituted cycloalkyl or substituted aromatic radical;
aᵢ is, for i = 1 to n, an integer greater or equal to 2;
R₂ is hydrogen or a hydrocarbon radical; and
n is an integer selected so that the HLB of said compound is comprised between 12 and 20
to provide antioxidant and antiviral properties and reduce gas permeability of the article, the article not being a vaginal device.

2. An article according to Claim 1, wherein R₁ is a substituted phenyl radical.

3. An article according to Claim 1, wherein R₁ is a phenyl radical substituted by at least one alkyl group.

4. An article according to Claim 1, wherein R₁ is a phenyl radical substituted in para position with a C₆₋₁₂ alkyl group.

5. An article according to any preceding claim, wherein the integers aᵢ and n are respectively equal to 2 or 3 and comprised between 6 and 20.

6. An article according to Claim 5, wherein the integer n is comprised between 8 and 15.

7. An article according to any preceding claim, wherein R₂ is hydrogen or alcohol.

8. An article according to any preceding claim, in which the polymer is polyethylene, polypropylene, polyvinyl chloride, polystyrene or polyamide.

9. An article according to any preceding claim, wherein the antiviral agent comprises from 2 to 10% of the compound.

10. An article according to Claim 9, wherein the antiviral agent comprises about 5% of the compound.

11. An article according to any one of Claims 1 to 10, in the form of a surgical glove, a finger stall, an apron, a bib, or a cap.

12. Use of a composition, which is an antioxidant and antiviral agent, in the manufacture of a polymeric material, for the purpose of plasticizing and reducing gas permeability of an article other than a vaginal device, made from or coated with a layer of the material, the composition having the general formula:
R₁ - O - ((CH₂))aᵢ - O)n - R₂
where:
R₁ is a substituted cycloalkyl or substituted aromatic radical;
aᵢ is, for i = 1 to n, an integer greater or equal to 2;
R₂ is hydrogen or a hydrocarbon radical; and
n is an integer selected so that the HLB of said compound is comprised between 12 and 20.

## Patentansprüche

1. Artikel, der aus einer plastifizierten polymeren Verbindung besteht oder mit einer Schicht einer solchen Verbindung beschichtet ist, wobei der Weichmacher, in einer Menge von mindestens 1 % der Verbindung, ein Mittel der allgemeinen Formel ist:
R₁ - O - ((CH₂)aᵢ -O)n - R₂
worin bedeuten:
R₁ ein substituiertes Cycloalkyl oder substituiertes aromatisches Radikal;
aᵢ für i = 1 bis n eine ganze Zahl von größer oder gleich 2 ist;
R₂ Wasserstoff oder ein Kohlenwasserstoffradikal ist; und
n eine ganze Zahl ist, die so ausgewählt ist, daß die HLB der Verbindung zwischen 12 und 20 liegt,
um Antioxidans- und antivirale Eigenschaften zu verleihen und die Gaspermeabilität des Artikels zu verringern, wobei der Artikel keine vaginale Einrichtung ist.

2. Artikel nach Anspruch 1, dadurch gekennzeichnet, daß R₁ ein substituiertes Phenylradikal ist.

3. Artikel nach Anspruch 1, dadurch gekennzeichnet, daß R₁ ein durch mindestens eine Alkylgruppe substituiertes Phenylradikal ist.

4. Artikel nach Anspruch 1, dadurch gekennzeichnet, daß R₁ ein in Parastellung mit einer C₆₋₁₂-Alkylgruppe substituiertes Phenylradikal ist.

5. Artikel nach einem der vorhergehenden Ansprüche, dadurch gekennzeichnet, daß die ganzen Zahlen aᵢ bzw. n gleich 2 oder 3 sind und zwischen 6 und 20 umfassen.

6. Artikel nach Anspruch 5, dadurch gekennzeichnet, daß die ganze Zahl n zwischen 8 und 15 liegt.

7. Artikel nach einem der vorhergehenden Ansprüche, dadurch gekennzeichnet, daß R₂ Wasserstoff oder Alkohol ist.

8. Artikel nach einem der vorhergehenden Ansprüche, dadurch gekennzeichnet, daß das Polymer Polyethylen, Polypropylen, Polyvinylchlorid, Polystyrol oder Polyamid ist.

9. Artikel nach einem der vorhergehenden Ansprüche, dadurch gekennzeichnet, daß das antivirale Agens 2 bis 10 % der Verbindung umfaßt.

10. Artikel nach Anspruch 9, dadurch gekennzeichnet, daß das antivirale Agens ca. 5 % der Verbindung umfaßt.

11. Artikel nach einem der Ansprüche 1 bis 10 in Form eines Einweghandschuhs, eines Fingerschutzes, einer Schürze, eines Latzes oder einer Kappe.

12. Verwendung einer Zusammensetzung, die ein Antioxidans und ein antivirales Mittel ist, zur Herstellung eines polymeren Materials zum Zwecke der Plastifizierung und der Verringerung der Gaspermeabilität eines von einer vaginalen Einrichtung verschiedenen Artikels, der hergestellt ist aus dem Material oder mit einer Schicht des Materials beschichtet ist, wobei die Zusammensetzung die allgemeinen Formel besitzt:
R₁ - O - ((CH₂)aᵢ - O)n - R₂
worin bedeuten:
R₁ ein substituiertes Cycloalkyl oder substituiertes aromatisches Radikal;
aᵢ für i = 1 bis n eine ganze Zahl von größer oder gleich 2 ist;
R₂ Wasserstoff oder ein Kohlenwasserstoffradikal ist; und
n eine ganze Zahl ist, die so ausgewählt ist, daß die HLB der Verbindung zwischen 12 und 20 liegt.

## Revendications

1. Article fabriqué à partir de ou revêtu d'une couche d'un composé polymère plastifié, le plastifiant comprenant, à raison d'au moins 1% du composé, un agent de la formule générale :
R₁ - O - ((CH₂)aᵢ - O)ₙ - R₂
dans laquelle :
R₁ est un cycloalkyle substitué ou un radical aromatique substitué ;
aᵢ est un entier supérieur ou égal à 2, avec i = 1 à n ;
R₂ est un hydrogène ou un radical d'hydrocarbure ; et
n est un entier choisi de telle sorte que l'équilibre hydrophile/lipophile dudit composé est compris entre 12 et 20 ;
afin d'obtenir des propriétés antioxydantes et antivirales et de réduire la perméabilité aux gaz de l'article, ledit article n'étant pas un dispositif vaginal.

2. Article selon la revendication 1, dans lequel R₁ est un radical phényle substitué.

3. Article selon la revendication 1, dans lequel R₁ est un radical phényle substitué par au moins un groupe alkyle.

4. Article selon la revendication 1, dans lequel R₁ est un radical phényle substitué en position para par un groupe alkyle en C₆-C₁₂.

5. Article selon l'une quelconque des revendications précédentes, dans lequel les entiers aᵢ et n sont respectivement égaux à 2 ou 3 et compris entre 6 et 20.

6. Article selon la revendication 5, dans lequel l'entier n est compris entre 8 et 15.

7. Article selon l'une quelconque des revendications précédentes, dans lequel R₂ est un hydrogène ou un alcool.

8. Article selon l'une quelconque des revendications précédentes, dans lequel le polymère est le polyéthylène, le polypropylène, le polychlorure de vinyle, le polystyrène ou le polyamide.

9. Article selon l'une quelconque des revendications précédentes, dans lequel l'agent antiviral représente de 2 à 10% du composé.

10. Article selon la revendication 9, dans lequel l'agent antiviral représente environ 5% du composé.

11. Article selon l'une quelconque des revendications 1 à 10, sous forme de gant chirurgical, de doigtier, de tablier, de bavette ou de coiffe.

12. Utilisation d'une composition, qui est un agent antioxydant ou antiviral, dans la fabrication d'un matériau polymère, dans le but de plastifier et de réduire la perméabilité aux gaz d'un article autre qu'un dispositif vaginal, fabriqué à partir de ou revêtu d'une couche du matériau, la composition ayant la formule générale :
R₁ - O -((CH₂)aᵢ - O)ₙ - R₂
dans laquelle :
R₁ est un cycloalkyle substitué ou un radical aromatique substitué ;
aᵢ est un entier supérieur ou égal à 2, avec i = 1 à n ;
R₂ est un hydrogène ou un radical d'hydrocarbure ; et
n est un entier choisi de telle sorte que l'équilibre hydrophile/lipophile dudit composé est compris entre 12 et 20.
